# EUROPEAN PATENT SPECIFICATION

(11) **EP 1 979 036 B1**
(45) Date of publication and mention of the grant of the patent: **04.09.2013**
(21) Application number: 07717904.2
(22) Date of filing: 08.01.2007
(51) Int. Cl.: A61M 25/01, A61F 2/06

(54) **A MEDICAL DELIVERY SYSTEM OF A MEDICALLY USEFUL PAYLOAD**
MEDIZINISCHES VERABREICHUNGSSYSTEM EINER MEDIZINISCHEN NUTZLAST
SYSTÈME D'ADMINISTRATION MÉDICALE ET PROCÉDÉ D'ADMINISTRATION DE CHARGE UTILE DU POINT DE VUE MÉDICAL

(30) Priority: 06.01.2006 US 757123 P
(43) Date of publication of application: 15.10.2008
(73) Proprietor: Cordis Corporation, Miami Lakes, Florida 33014 (US)
(72) Inventor: BURGERMEISTER, Robert, Bridgewater, New Jersey 08807 (US); KREVER, Mathew, Warren, New Jersey 07059 (US); FERRARA, Paul, Green Brook, New Jersey 08812 (US); DE CAPRIO, Fernando, St. Paul, Minnesota 55105 (US); SMELSER, Ricci, Dayle, Maple Lake, MN 55358 (US); BEYREIS, Randall, James, Corcoran, MN 55340 (US); HATCHER, Brady, Jon, Rogers, MN 55374 (US)
(74) Representative: Small, Gary James
(86) International application number: PCT/US2007/060243
(87) International publication number: WO 2007/082189

(56) References cited:
- EP-A2- 0 508 473
- EP-A2- 1 101 455
- WO-A-96/39999
- WO-A-03/002037
- US-A- 5 041 126
- US-A1- 2001 049 549
- US-A1- 2005 049 667
- US-B1- 6 187 034

## Description

### FIELD OF THE INVENTION

The present disclosure concerns a delivery system for delivering a medically useful payload through a channel in the patient's body, such as the vasculature or a lumen, to a site of interest. The medically useful payload may be a therapeutic device, such as a stent, or it may be a diagnostic tool, such as a camera. Owing to its structural or shape attributes, the presently-inventive delivery system is well suited for carrying medical payloads to and through vessel curvature and to branched regions (e.g., bifurcations) in same. Also, the device is well-suited to traveling through a vessel over a guiding element, such as a guidewire, which itself exhibits curvature.

### BACKGROUND OF THE INVENTION

Diseases of the vasculature, such as stenoses, strictures or aneurysms in blood vessels and other body vessels can be treated or diagnosed by locating a payload, such as a stent, graft, imaging device, or the like, at the site of disease. Such payload can be carried to the site of implantation by a delivery device having a catheter for carrying and activating the payload. The catheters can be expected to carry the payload over a relatively long distance, often from an incision in the patient's groin area, through the vasculature, to a location where action is required. For example, a site in the vicinity of the patient's heart may be the target for payload deployment.

From incision to deployment site, the path is defined by the interior of a vessel that the catheter must travel. The vessel may have segments that are difficult to traverse. Curves or bifurcations in vessels exemplify two particular kinds of segments that can present such difficulties. Likewise, the deployment site may be curved, or a bifurcation may be present at the site of deployment.

A bifurcation in a vessel is a location where the vessel divides into two branches or parts. Vascular bifurcations generally have circumferential asymmetry. That is, bifurcated vessels generally exhibit asymmetry around their circumference at the point where the main vessel divides into two branches. Thus, the opening in the side branch vessel where the side branch vessel joins the main branch vessel may be asymmetrical. The side branch vessel may join the main branch vessel at an oblique angle; which may contribute to the asymmetry of the bifurcation cross-section.

One kind of prior art bifurcation delivery device employs multiple guidewires and/or the clinician to orient and manipulate the device relative to the bifurcation. For example, attempts have been made to accomplish this solely through the use of two wires or wire-like elements (one in each branch of bifurcation) to force rotation of the device to match the vessel anatomy. This approach has shortcomings. First, by requiring delivery of the medical device to the location of the bifurcation over two wires (for substantially the entire delivery), the chance of wire wrapping is greatly increased. This prevents complete delivery of the device and can result in the clinician having to withdraw a wire and rewire the vessels, causing significant procedural delay and patient risk. Second, reliance on two wires for device orientation is typically insufficient to guarantee full and proper alignment of the entire medical device with the side branch ostium (particularly the portion of the device proximal to the carina (or apex) of the bifurcation). Even when both branches of the bifurcation are wired and the medical device is seated on the carina, the wires are typically not able to exert enough rotational influence on the device to align the whole length of the payload.

In any event, carrying the payload through a vessel curvature, a bifurcation, or otherwise deploying the payload at such locations can present challenges in terms of traversing or accessing the site. Furthermore, where the payload needs to be in a specific orientation (such as for maximizing the therapeutic effect or diagnostic purpose of the payload), achieving the desired orientation in such curvature or bifurcation presents yet another challenge to the person of skill in the art.

U.S. Patent no. 6,544,218, entitled "Catheter With Biased Shaft" is disclosed as a reference of interest.
European patent publication no. EP 0508473 discloses an endovascular graft having bifurcation and an apparatus and method for deploying the same.
US patent publication no. US2001/0049549 discloses a marker device for rotationally orienting a stent delivery system prior to deploying a curved self-expanding stent.
US patent no. 5,041,126 discloses a wire stent for insertion and expansion into a passageway which comprises a plurality of curved sections that are formed into a generally circular configuration.

### SUMMARY OF THE INVENTION

The present invention as defined by claim 1 is directed to a system for the delivery of a medically useful payload to a target site within a patient's body. By way of example, the system may be a flexible catheter, and the medically useful payload may be a stent or atherectomy device. In these instances, the medically useful payload is delivered to a site of disease within a blood vessel of a patient. In yet other examples the medically useful payload may be a camera, a light, or both, which can be carried to a site where observation is warranted for purposes of making a medical diagnosis. In one aspect of the present invention, an orienting region exhibiting a curved shape is located at a distal end of the delivery device. The curved shape facilitates rotation of the payload into a desired position during delivery according to its preferential orientation. That is, the orienting distal region is curved or bent in a preferred direction that causes the device to rotate in accordance with the shape of the vessel or guidewire (if possessing a curved segment) on which the delivery device may be tracked in order to achieve desired orientation of the payload.

Thus, the delivery device of the present invention is adapted to deliver medical payloads (such as stents) to vessels that are curved and/or bifurcated, or other vessel configurations, such as those with eccentric lesions that are better serviced by deploying oriented devices. That is, there are occasions where the device should be oriented in a specific fashion relative to the bifurcation, curvature, or other vessel feature, or even oriented in response to a bend in the guidewire. Such orientation can be achieved with the present invention.

In the present invention, the orienting member having a curved shape is distally located on the delivery device and is coupled thereto to allow a degree of torsional movement of the orienting member, relative to other parts of the device. That is, the orienting member is attached to the delivery device in a manner that allows the orienting member to rotate as necessary to orient the member and conform the distal end of the device, where the member is located, to the shape of the vasculature, in order to deploy or carry the payload so the payload can be properly oriented.

In a further aspect of the present invention, the payload can be co-located with the orienting member. For example, a stent can be positioned in or over the orienting member. In an alternative arrangement, the payload is not co-located with the orienting member, yet is coupled to the orienting member through sufficient intervening structure so as to undergo rotation in response to the orientation of the orienting member.

A specific aspect of the present invention includes a device for carrying a medically useful payload positioned upon the device to a location of interest relative to a non-linear path in a body lumen. The device comprises an elongate intralumenal member having proximal and distal end portions. The intralumenal member is sized and dimensioned to travel to a location of interest in a body lumen. The device further includes an orienting member positioned along the distal end portion of the elongate intralumenal member. The orienting member exhibits a curved shape and is configured to rotate in response to the non-linear path.

The curved shape imparted to the orienting member of the present invention facilitates orientation of the device as it travels through (1) curves or bifurcations in the vessel, (2) curves or bends in the guidewire, or (3) other eccentricities located within the vessel that create a curved path. So long as the orienting member possesses a sufficient degree of freedom to rotate, it will assume the path of least resistance in the course of its travels, and thereby rotate/orient itself to conform to the curve in the vessel in a unique, repeatable and predictable manner. Thus, by linking or associating a payload with the orienting member in a known relative position, orientation of the payload can be attained as a result of the rotational action exhibited by the orienting member.

Aside from being adapted to pass relatively easily through bends and curves in the vasculature, the self-orienting member can be used in a number of beneficial ways. Stents deployed at the site of or in the vicinity of a bifurcation may have asymmetrical design features intended to conform to the bifurcation, and in particular, the side branch ostium. Such stents must be deployed in the proper orientation, a result that can be obtained by coupling such stents to the orienting member, and then allowing the member to orient itself in the vessel. Likewise, a camera or other diagnostic tool, such as an ultrasound transducer (IVUS), pressure transducer, infrared sensor, endoscope lens coupled to the orienting member could be properly oriented as a result of orienting member orientation. Furthermore, the self-orienting nature is useful where the curve, so to speak, is imparted by the guidewire that passes through the catheter. For instance, the orienting member may travel over a guide wire passed into a bifurcation side branch, allowing a stent to be deployed, in its proper orientation, in the side branch. In yet another example, a guidewire having a pre-bent or curved section can be used to effect orientation of the orienting member in situations where vessel characteristics are not of an orientation-producing nature. In other words, by positioning the bend in the guidewire at the desired location, the orienting member will orient itself as it traverses the bend. This arrangement is advantageous where it is desirable to achieve orientation in a relatively straight vessel segment. In any event, with these arrangements, rotation of the orienting member for positioning of payload, whether for deployment or other medically useful purpose is facilitated. Further, it should be understood that with the orienting member of the present invention, it is not just the payload that is properly oriented. For example, in the case of a bifurcated vessel, the side branch guidewire exit port can be oriented to face the ostium of the side branch vessel. In other words, as the orienting member rotates, the side branch guidewire exit port aligns according to the orienting member orientation, with the side branch guidewire element facing the side branch ostium. This arrangement makes it possible for the orienting member to properly orient to the side branch anatomy when the device is seated at the carina of the bifurcation. This arrangement also makes it easier for the side branch guide wire to be advanced out of the delivery catheter and into the side branch.

The inducement of a curved shape in the orienting member along the distal end of the catheter may be made at any time before or during the delivery process, but is preferably done prior to advancement of the device to the bifurcation. Accordingly, the delivery system may include an element capable of inducing a curved shape along the orienting member assist in controlling the orientation of the catheter 10 and payload. The elements may be active component elements or passive component elements.

Chemical, electrical/thermal or mechanical means can be used to actively modify the orienting member so the orienting member can be transversely displaced, resulting in a preferred curvature. For example, the orienting member may be provided with a curve by the manual placement or displacement of a bent or bendable member within the orienting member.

Passive components may be part of or incorporated into the delivery device or orienting member to allow the delivery device or orienting member to be plastically deformed into a curved shape.

### BRIEF DESCRIPTION OF THE DRAWINGS

Figure 1 is a perspective view illustrating a delivery device according to one embodiment of the present invention.
Figure 2 is a cross-sectional view illustrating the delivery device shown in Figure 1 according to one embodiment of the present invention.
Figure 3 is a cross-sectional view illustrating the delivery device shown in Figure 1 according to one embodiment of the present invention.
Figure 4A is a cross-sectional view illustrating another aspect of the present invention.
Figure 4B is a cross-sectional view illustrating another aspect of the present invention.
Figure 5A is a side view illustrating the distal end of the delivery system, particularly the delivery catheter, having an orienting member attached along the distal end according to one embodiment of the present invention.
Figure 5B is a cross-sectional view illustrating the distal end of the delivery system, particularly the delivery catheter, having an orienting member attached along the distal end according to one embodiment of the present invention.
Figure 6A is a perspective view illustrating a delivery device having a shaped balloon (in an un-inflated configuration) located along the orienting member according to one embodiment of the present invention.
Figure 6B is a perspective view illustrating a delivery device having a shaped balloon (in an inflated configuration) located along the orienting member according to one embodiment of the present invention.
Figure 7A is a schematic side view of a catheter having an orienting member that includes active elements capable of inducing a curved shape to the orienting member upon activation.
Figure 7B is a section view of a catheter having an orienting member that includes active elements capable of inducing a curved shape to the orienting member upon activation.
Figure 7C is a schematic side view of a catheter having an orienting member that includes active elements that induced a curved shape to the orienting member after activation.
Figure 8A is a magnified side view of a mechanical type active element according to one embodiment of the present invention.
Figure 8B. is a magnified section view of a mechanical type active element 61 according to one embodiment of the present invention.
Figure 8C is a magnified side view of a mechanical type active element according to one embodiment of the present invention.
Figure 8D is a magnified side section view of a flexible tip guidewire used as an active element 61 according to one embodiment of the present invention.
Figure 9A is a side view of a delivery device illustrating passive biasing elements incorporated into the orienting member according to one embodiment of the present invention.
Figure 9B is a section view of a delivery device illustrating passive biasing elements incorporated into the orienting member according to one embodiment of the present invention.
Figure 10A shows a catheter delivery system having a curved shape with a secondary lumen on the outside radius of the orienting member, also referred to as a greater curve bend, according to one embodiment of the present invention.
Figure 10B shows a catheter delivery device having a curved shape with the secondary lumen on the inside radius of the orienting member, which is also sometimes referred to as a lesser curve bend, according to one embodiment of the present invention.
Figure 11A shows a catheter delivery system, with a pre-bent biasing wire, having a curved shape with a secondary lumen on the outside radius of the orienting member, also referred to as a greater curve bend, according to one embodiment of the present invention.
Figure 11B shows a catheter delivery device, with a pre-bent biasing wire, having a curved shape with the secondary lumen on the inside radius of the orienting member, which is also sometimes referred to as a lesser curve bend, according to one embodiment of the present invention.
Figures 12A illustrates a vessel anatomy having a main branch bend and a side branch off the greater curve (convex side) of the main branch bend according to one embodiment of the present invention.
Figures 12B illustrates a vessel anatomy having a main branch bend and a side branch off the lesser curve (concave) of the main branch bend according to one embodiment of the present invention.
Figure 13A shows a guidewire deployed in a main branch vessel having a greater curve anatomy according to one embodiment of the present invention.
Figure 13B shows a guidewire deployed in a main branch vessel having a lesser curve anatomy according to one embodiment of the present invention.
Figure 14A shows a delivery catheter deployed in a main branch vessel having a greater curve anatomy along two guidewires according to one embodiment of the present invention.
Figure 14B shows a delivery catheter deployed in a main branch vessel having a lesser curve anatomy along two guidewires according to one embodiment of the present invention.

### DETAILED DESCRIPTION OF THE INVENTION

This invention describes a novel construction for designing the distal portion of a medical device delivery system. The delivery system can track the medical device towards a target location on a single wire, and pre-orient the payload of the delivery system relative to the target location. This design facilitates rotation of a payload into position for use. To accomplish this rotation, a curved shape is induced on the orienting member of the delivery system prior to advancement of the device to the target location. As an example in this embodiment, the curved shape should be chosen such that the curvature of the device is approximately matched to curvature in the vicinity of the target location. As the device rotates, the curve of the device aligns with the curve of the vessel, orienting the payload into the desired position.

In one particular embodiment, this invention describes a novel construction for designing the distal portion of a medical device delivery system, such as a stent delivery system (SDS). The delivery system can track the medical device towards the bifurcation on a single wire, and pre-orient the side branch features of the delivery system and device towards the side branch ostium. This design facilitates rotation of a bifurcation device into position for deployment. To accomplish this rotation, a curved shape is induced on the orienting member prior to advancement of the device to the bifurcation. As an example in this embodiment, the curved shape should be chosen such that the curvature of the device is approximately matched to curvature in the vicinity of the ostium, with the side branch guidewire exit port facing the ostium of the side branch vessel. As the device rotates, the curve of the device matches the curve of the main branch vessel and the side branch device element will end up facing in the direction of the side branch ostium. This ensures that the device orients fully to the side branch anatomy once both branches are wired and the device is seated at the carina of the bifurcation. The curved shape of the device could also be used to match the curve of the side branch vessel if the main branch vessel does not have pronounced curvature.

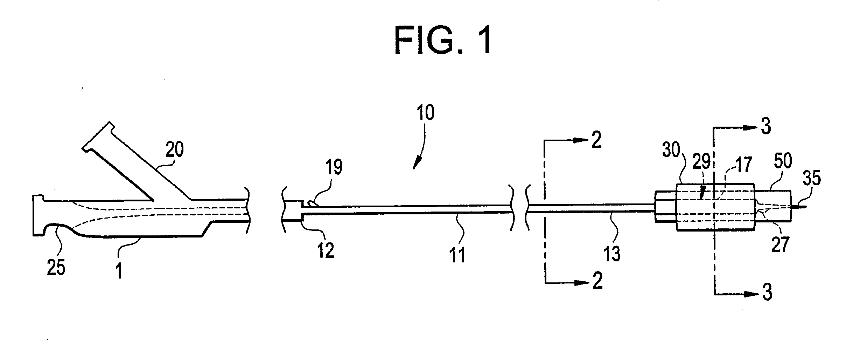

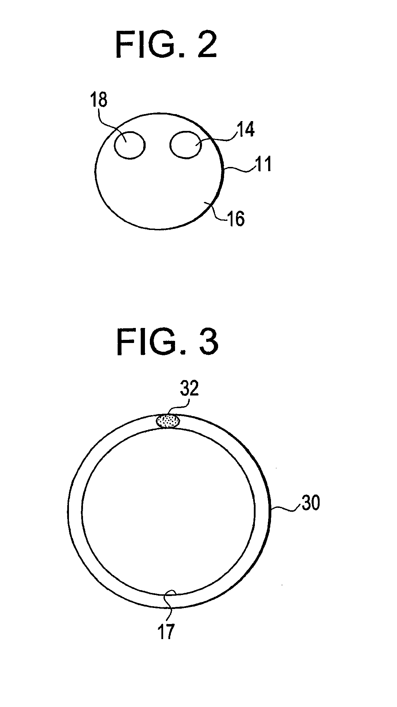

FIGS. 1 and 2 illustrate a delivery system, including catheter 10 of the kind suitable for treating bifurcations in which the payload is a balloon expandable stent. Catheter 10 includes an inflatable balloon and therefore is useful for deploying a balloon expandable stent, although it should be understood that the delivery system described herein could be employed with a self expanding stent, obviating the need for an inflation balloon and lumen to transport inflation fluid, it should be noted that if the payload were a self expanding stent, such as one manufactured of nitinol material, then the device would require a outer restraining sheath positioned over the stent. Catheter 10 generally comprises an elongated catheter shaft 11 having a proximal end 12, a distal end 13. At least one guidewire lumen 14 is adapted to receive and pass a guidewire, though a second guidewire lumen 18 is depicted here. An inflation port 20 at the proximal end of catheter shaft 11 has an opening for receiving an inflation fluid from an external source. The inflation port is in fluid communication with the open annular space 16 present within shaft 11. Guidewire entry port 25 is found at the proximal device end, and can be in communication with guidewire lumen 14 or 18 that are discussed below. An RX guidewire lumen port 19 is located between proximal and distal ends 12 and 13 as shown in Figure 1. RX guidewire lumen port 19 is in communication with the other of the guidewire lumens 14 or 18.

In the embodiment of FIG. 1, the shaft 11 is a multiple-lumen shaft, typically extruded or otherwise formed with at least one guidewire lumen 14, and possibly a second guidewire lumen 18 present in annular space 16. Annular space is sealed on its exterior by wall of shaft 11 and therefore serves as the conduit for transferring the balloon inflation fluid. Here, where the device is intended for the delivery of a stent, at the site of a bifurcation, it is advantageous, though not entirely necessary, to employ a catheter that can receive and pass at least two guidewires 32 and 33. The catheter shaft is provided with lumens for main branch and side branch guidewires, such lumens designated 14 and 18, respectively.

An inflatable balloon 17 is disposed on a distal section of catheter shaft 11, having a proximal end and a distal end secured and sealed to the shaft 11. The opening in the balloon at its proximal end is in fluid communication with annular space 16 so that the balloon can receive and retain the inflation fluid as it flows from the external source, through inflation port 20, through annular space 16, and into balloon 17. A stent 30 is mounted over the balloon 17. See Fig. 3. Also shown is a side branch guidewire 32, indicating that the payload tracks the side branch guidewire and thus payload deployment is to take place adjacent to a side branch ostium of a bifurcated vessel. By no means is this depiction intended to restrict the scope of the present application in any way, as the payload could have been depicted as tracking the main branch guidewire, i.e., -- intended for deployment in the main branch of the bifurcation.

An orienting member 50 is located at the distal end of the catheter 10. Orienting member 50 is mounted at the distal end of shaft 11. In the inventive arrangement, the proximal end of shaft 11 is constructed of a material that exhibits sufficiently high torsional flexibility so that the orienting member portion of the catheter is able to undergo the rotation necessary to orient the device. Employing an elastomeric material to construct the shaft in the area proximal to the orienting member 50 is one way to attain this result. In other arrangements, dimensions of shaft 11 can be varied in order to impart torsional flexibility in the region where the orienting member joins to the catheter. For example, the wall thickness of shaft 11 can be reduced in the area proximal to where the shaft 11 joins to the orienting member 50, or alternatively, the diameter of the shaft 11 in this region can be reduced. In yet another arrangement, the orienting member is butt welded to the end of one of the guidewire lumens 14 or 18.

Distal exit ports 27 and 29 for main branch and side branch guide wires, respectively, are located at approximately the mid-portion of the distal payload for the side branch guidewire (not shown) and the catheter tip (35) for the main branch guidewire.

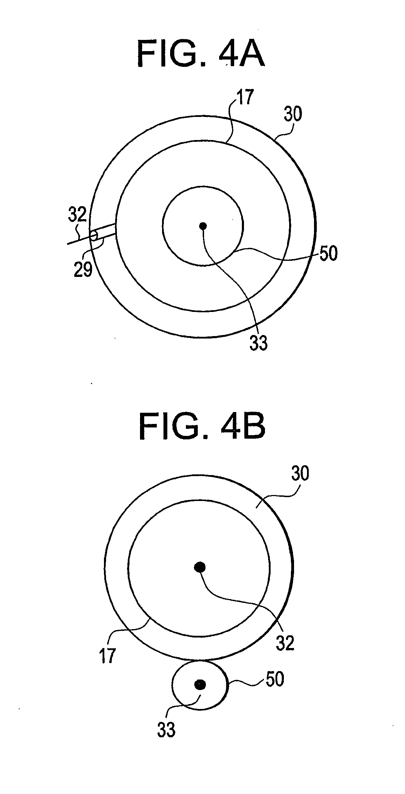

As shown in FIG. 4A, the orienting member 50 is coaxially arranged with the main branch guide wire 33, that is, with the guidewire arranged coaxially within the orienting member 50. Orienting member 50 extends within balloon 17 and is co-located therewith for at least a portion of its length with balloon 17 (FIG. 1), with the stent 30 being carried over the balloon and orienting member portion. A portion of the orienting member 50 extends sufficiently distal to the payload in order to orient the device and payload for deployment.

According to another embodiment of the present invention, as shown in FIG. 4B, the orienting member 50 is coaxially arranged with the main branch guide wire 33, that is, with the guidewire arranged coaxially within the orienting member 50. Balloon 17 is coaxially located with the side branch guide wire 32 with the stent 30 being carried over the balloon. A portion of the orienting member 50 extends sufficiently distal to the payload in order to orient the device and payload for deployment.

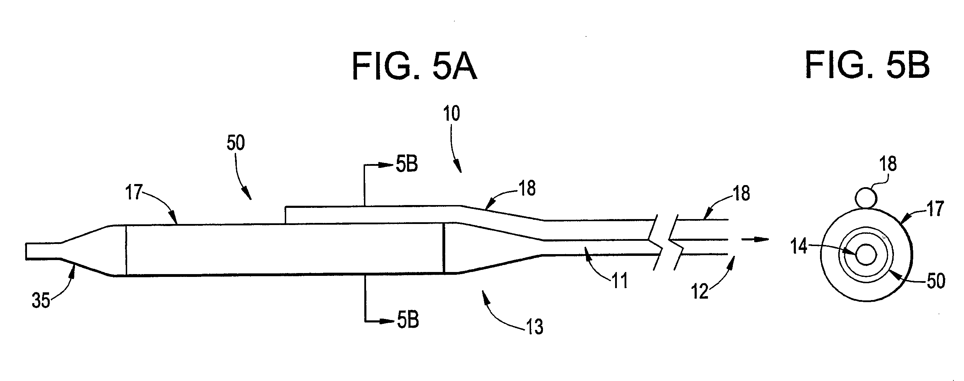

Figures 5A and 5B illustrate side and section views, respectively, of the distal end of the delivery system, particularly catheter 10 having orienting member 50 attached along the distal end 13 according to one embodiment of the present invention. It should be noted that the medical device (stent) has been removed for clarity, and that the device depicted in these figures does not yet have the curved shape induced along the distal end portion.

As described above, catheter 10 comprises an outer body, catheter shaft 11, having proximal 12 and distal 13 ends, and an inner body or bodies forming guidewire lumens 14, 18, etc. The distal end may also have a distal tip 35 to assist the delivery system when tracking over a guidewire and/or navigating through the vasculature.

An expandable member, balloon 17, is attached along the distal end of catheter 10, and the secondary guidewire lumen 18 is located along the outer surface of the expandable member 17.

A hub may be integrated in the proximal shaft to allow the catheter 11 to be attached to a handle, inflation port, or similar device along the proximal end 12. In one embodiment of the invention, the catheter 10 is made to track over a guidewire placed through the vasculature to the target location. Any methods known in the art for tracking the catheter 10 over a guidewire may be used, including an Over the Wire (OTW) type device or a Rapid Exchange device. Accordingly, the catheter 10 may have an OTW (Over the Wire) port or an Rx (Rapid Exchange) port respectively.

As earlier described, the purpose of the catheter 10 is to deliver and orient a medical device to the target location in a vessel. A stent has been described as the medical device to be oriented in a particular manner at a vessel bifurcation for the purpose of example. However, one of skill in the art would understand that other medical devices that can be incorporated into the distal end of the delivery catheter can also be delivered and oriented, including but not limited to medical balloons, cameras, sensors, drug delivery devices, and various lumens.

One key concept of the present invention is the inducement of a curved shape in the orienting member 50 along the distal end of the catheter 10. The curved shape may be made at any time before or during the delivery process, but is preferably done prior to advancement of the device to the target location. Accordingly, the delivery system may include an element capable of inducing a curved shape along the orienting member 50 to assist in controlling the orientation of the catheter 10 and payload. The elements may be active component elements or passive component elements.

Active components include components that can be shaped into a curved shape after introduction into the vasculature. In a preferred embodiment, active components can enter the vasculature having a relatively straight longitudinal configuration, such as the device illustrated in Figure 5A, and be bent into the desired shape at a subsequent time, preferably once the device is near the desired location. However, this sequence description is not meant to limit the scope of the invention, and one of skill in the art would understand that the active components could be shaped into a curve prior to entering the vasculature.

There are several methods that can be employed to actively induce the curved shape along the distal end of the catheter 10 and are presented here for the purpose of example. One of skill in the art would understand that other methods might also be employed.

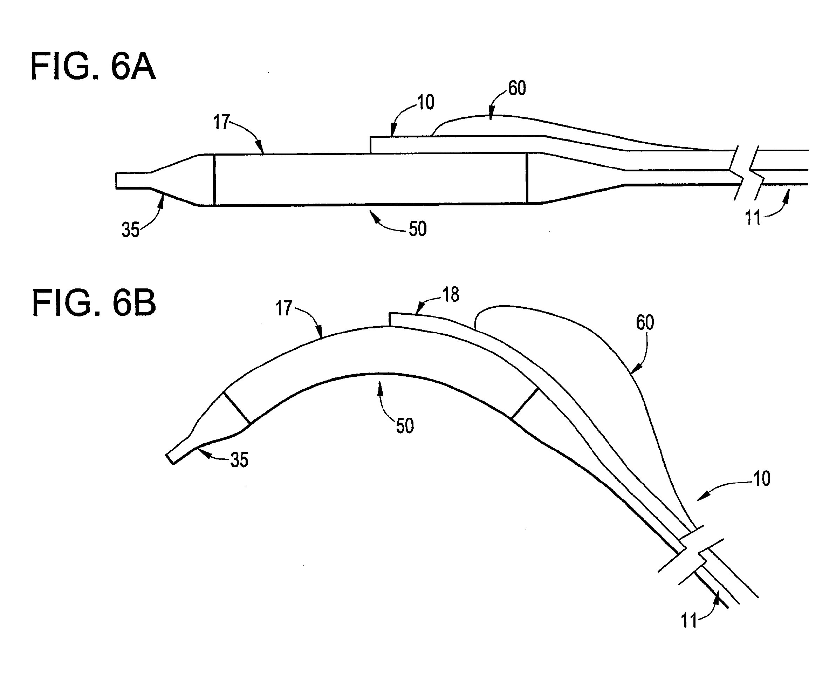

One method that can be used to induce a curved shape utilizes hydraulics and a pressure chamber that assumes a curved shape when filled. For example a curved or shaped medical balloon may be incorporated into the orienting member 50 or along catheter 10. Figure 6A and 6B are perspective views of a catheter 10 having a shaped balloon 60 located along the orienting member 50, separate from balloon 17.

The balloon 60 is shown in the un-inflated configuration in Figure 6A, with the catheter 10 and orienting member 50 assuming a relatively straight position. During delivery the balloon 60 remains relatively straight and flexible in an un-inflated, constrained and wrapped configuration. To impart the curved shape along the orienting member 50, a fluid is introduced into the balloon 60 through the delivery catheter 10, increasing the balloon's internal pressure and ultimately filling the balloon 60 until the balloon 60 assumes is curved shape, as shown in Figure 6B. The inflated balloon is strong and rigid enough to deflect the orienting member 50 into the desired curved shape. The fluid may be compressible or incompressible, but is preferably a substantially incompressible biologically compatible fluid such as saline.

In another embodiment of the invention, the orienting member 50 includes at least one element that is capable of changing shape upon the introduction of some type of energy. This energy source may come in the form of mechanical, electrical, chemical, thermal or magnetic energy.

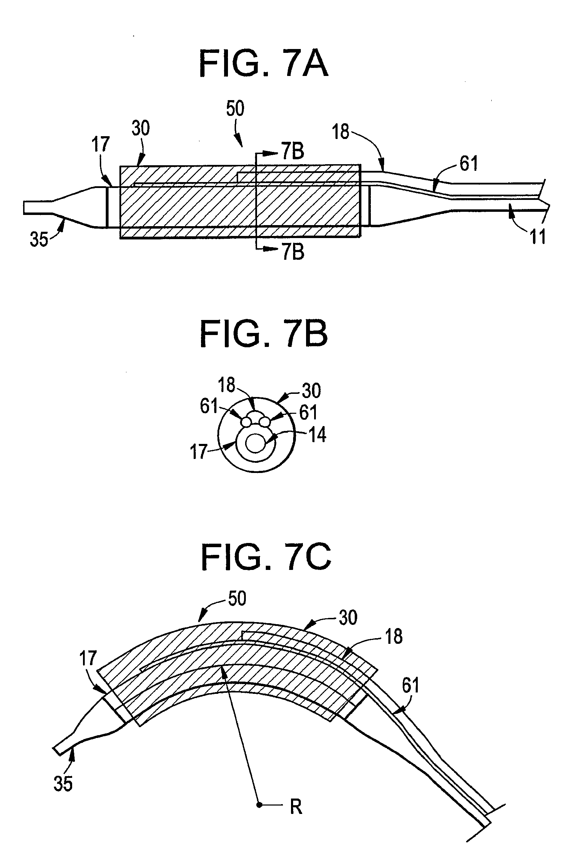

Figure 7A and 7B are side and section views of catheter 10 having an orienting member 50 that includes active elements 61 capable of inducing a curved shape to the orienting member 50 upon activation. Figure 7C is a side view illustrating the orienting member 50 in a curved configuration after activation of active elements 61.

Active elements 61 are typically activated by a clinician or technician manipulating or triggering an operable component associated with the active element. In many cases, the operable component is located along the proximal end of catheter 10. Accordingly, some embodiments of the delivery catheter 10 utilizing active elements 61 have a communication means between the operable component and the active element 61. In other embodiments of the invention, the active element 61 itself, or a component part of the active element may traverse through a secondary lumen in the catheter 10 to the proximal end portion 12. In still other embodiments of the invention, the active element 61 is capable of automatically activating by changes in environmental conditions. In this latter case, it may not be necessary for the active component to have a manually operable component along the proximal end portion 12 of catheter 10.

In one embodiment of the invention the active element 61 is a mechanical element or elements that changes the shape of the orienting member 50 through a application of relative motion or force transmitted by a pull wire or similarly configured communication member attached to a proximally located operable component. These elements 61 may include wires, deflectable tubes, deflectable catheters, or deflectable tip guidewires (for example the Cordis Steer-it TM guidewire) and are generally known in the art of deflectable tip catheters and guidewires.

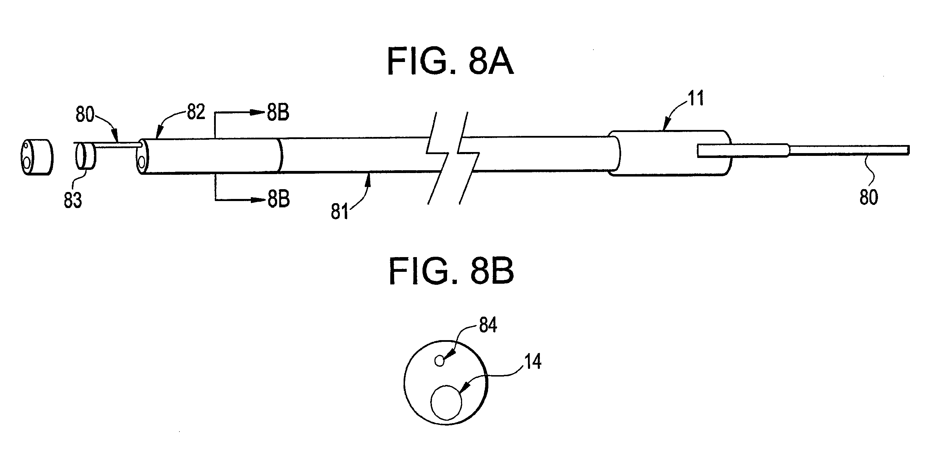

Figure 8A and 8B are a magnified side and section views of another mechanical type active element 61 according to one embodiment of the present invention. The mechanical active element 61 comprises a semi-compliant element 81 attached along the distal end portion of catheter shaft 11. A flexible element 82 is fixedly attached to the distal end of the semi-compliant element 81, and is of sufficient flexibility to bend when put under a compressive loading condition to provide the desired curvature in the orienting member 50. A pull wire 80 is attached to an anchor 83 located along the distal end of the flexible member 82, and travels proximally through pull wire lumen 84 and catheter shaft 11 to an operable component located along the proximal end of catheter 10. The pull wire lumen 84 is off center, which causes the flexible member 82 to deflect in a particular direction. That is, the offset pull wire lumen caused the flexible member 82 to curve such that the inside radius or lesser curve is on the side of the pull wire lumen 84.

The active elements 61 are located within or along the orienting member 50, and remain relatively straight and flexible when introduced into the vasculature and advanced to the desired location.

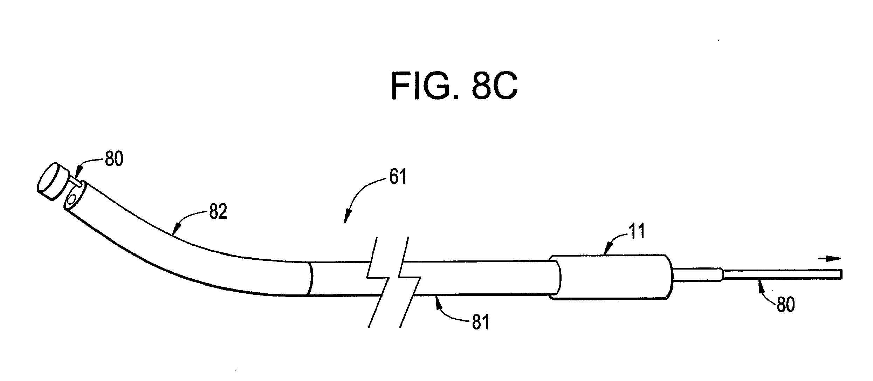

When the clinician desires to induce the curved shape he imparts mechanical force or motion to the pull wire 80 at the proximal end of the delivery catheter 10, which translates this energy into motion along the deflectable distal end portion to the deflectable element 82 associated with orienting member 50. This deflection bends the orienting member 50 to assume the curved shape. Figure 8C is a magnified side view of the mechanical active element 61 according to one embodiment.of the present invention when deflected into a curved shape.

In another embodiment of the present invention, a deflectable tip guidewire may also be suitable for use in as an active element 61 in conjunction with the orienting member 50. A similar guidewire concept is disclosed in US Patent No. 7,128,718 entitled Guidewire With Deflectable Tip. The disclosed deflectable guidewire is bi-directional and has a deflectable distal tip that comprises a longitudinal hypotube and a spring coil attached to the distal end of the hypotube. The deflectable guidewire also includes a longitudinally movable deflection member that is attached to the distal end of the spring coil and a tip retaining member that extends from the distal end of the hypotube to the distal end of the spring coil for providing very precise deflection of the distal tip.

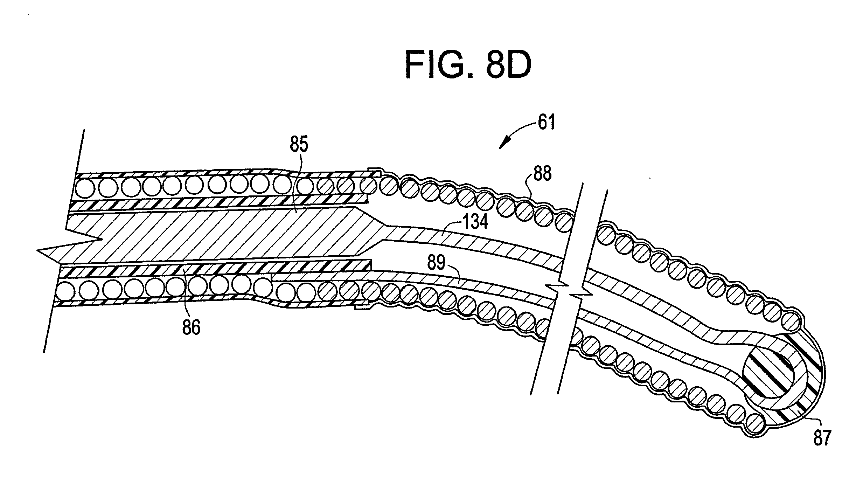

Figure 8D is a magnified side section view of a flexible tip guidewire used as an active element 61 according to one embodiment of the present invention. The actively bendable guidewire comprises an elongated hypotube 86, a helical coil 88 attached to and extending from the distal end of the hypotube 86. The helical coil 88 is preferably formed from platinum tungsten with the proximal turns being wound such that adjacent turns of the proximal portion are in contact with each other.

While the preferred embodiment of the present invention includes the helical coil 88, this element may take the form of any flexible cylindrical member, such as for example a thin metallic tube with or without portions of the tube removed by, for example laser cutting, so as to form a very flexible cylindrical member. An elongated, deflection member 85 extends from the proximal end of the control handle through the hypotube 86 and through the helical coil 88, and is connected into an attachment member, or rounded bead 87, which is disposed at the distal tip of the helical coil 88. In addition, a retaining ribbon 89 is connected to the distal end of the hypotube 86 and is also connected to the rounded bead 87.

In operation the distal tip of the active element 61 is normally biased into a downwardly curved position as illustrated in FIG. 8D, because of the curve of the pre-shaped deflection ribbon 134 and the retaining ribbon 89. When an operable component along the proximal end of the catheter 10 is moved distally, the deflection member 85 will be moved distally thereby causing the deflection ribbon 134 to move in a distal direction. As the deflection ribbon is moved distally, a force is applied to the top portion of the rounded bead 87. The retaining ribbon 89 is attached to the lower portion of the bead 87 to thereby maintain the bead at a fixed distance from the distal end of the hypotube 86. As the deflection ribbon 134 is moved to the right, the tip of the guidewire is caused to deflect downwardly to a maximum position of deflection.

In another embodiment of the invention, the orienting member 50 includes at least one active element 61 that changes shape corresponding to a change in electrical potential or current. Electrically active elements 61 that change shape under these conditions are known in the art, and include piezoelectrics, bimetallic strips with resistive heating elements and MEMS (electro-mechanical actuator) devices. Electrically active elements 61 may also include materials that exhibit shape memory properties under the influence of electric potentials. One such material known in the art is a family of conductive polymers that contract when the electrical potential is increased, and relax or elongate when depolarized. The contraction/relaxation of these elements may be used to locally cause a mechanical element to displace, resulting a bend or curve induced into the orienting member 50.

When the clinician desires to induce the curved shape he allows electrical energy to flow to the electrically active element 61. This may be, for example, by closing a switch at the proximal end 12 of catheter 10. This change is electrical potential or current causes the electrically active element 61 to change shape or deflect. This deflection bends the orienting member 50.

In still another embodiment of the invention, the orienting member 50 has at least one active element 61 that changes shape corresponding to a change in a magnetic field. The magnetic field may be internal to the body or vasculature, or external. One particular type of material for this type of application is a magnetostrictive material. Magnetostriction is the changing of a material's physical dimensions in response to changing its magnetization. In other words, a magnetostrictive material will convert magnetic energy into kinetic energy and change shape when it is subjected to a magnetic field. One brand name of a magnetostrictive material is Terfenol™

When the clinician desires to induce the curved shape he subjects the magnetostrictive active element 61 to a magnetic field. This magnetic field causes the magnetostrictive element 61 to convert the magnetic energy to kinetic energy and change shape. This deflection bends the orienting member 50 to assume the curved shape.

In other embodiments, the catheter 10, particularly orienting member 50 has ferrous or ferromagnetic portion or element 61 and responds to an external magnetic field. Accordingly, the orienting member 50 may bend toward the attractive magnetic field, or bend away from a repulsive magnetic field, causing a similar bend in the catheter 10.

The orienting member 50 may also be actively bent by forces exerted by a chemically responsive element 61 that changes shape corresponding to a change in local chemistry. This change may be caused by a chemical reaction or change in chemical concentration in the chemically active element 61. In either case the chemically responsive element 61 may swell or lengthen and change shape in response to the change in local chemistry. One of skill in the art would understand that this shape change or swelling may directly impart a curve in the orienting member 50. Similarly, the shape change or swelling may indirectly, through local manipulation of a mechanically active element, impart a curve in the orienting member 50.

Similarly, forces exerted by a thermally responsive active element 61 may also actively manipulate the orienting member 50. In these embodiments, the change in local temperature may be a result of placing the device into a warm vessel, or alternatively, changing the local temperature by introducing a hot or cold medium. In one particular embodiment, the thermally active element 61 is in fluid communication to an operable component located along the proximal end portion of catheter 10 through a channel. The clinician may initiate the flow of medium through the catheter channel to the thermally active element 61, causing the thermally active element to change shape and curve or bend the orienting member 50.

In one embodiment, a thermally responsive active element 61 may change shape corresponding to a change in temperature. For example, the material may undergo a change in shape as a result in the increase or decrease in local temperature.

In another embodiment the thermally responsive active element 61 has components with different coefficients of thermal expansion - for example a bimetallic strip. When the element is introduced into various thermal environments, the different components respond differently, and expand at different rates. This will cause the thermally responsive active element 61 to change shape or bend. This deflection bends the orienting member 50 to assume the curved shape. In addition, materials having anisotropic thermal expansion properties may be used to impart a curvature along the orienting member 50. One of skill in the art would understand that this shape change through thermal expansion may directly impart a curve in the orienting member 50. Similarly, the shape change through thermal expansion may indirectly, through local manipulation of a mechanically active element, impart a curve in the orienting member 50.

Thermally responsive active elements 61 may also undergo a phase change, causing the material to exhibit shape memory or super elastic characteristics. One such material is Nitinol.

Nitinol is utilized in a wide variety of applications, including medical device applications as described above. Nitinol or NiTi alloys are widely utilized in the fabrication or construction of medical devices for a number of reasons; including its biomechanical compatibility, its biocompatibility, its fatigue resistance, its kink resistance, its uniform plastic deformation, its magnetic resonance imaging compatibility, its ability to exert constant and gentle outward pressure, its dynamic interference, its thermal deployment capability, its elastic deployment capability, its hysteresis characteristics, and is moderately radiopaque.

Nitinol, as described above, exhibits shape memory and/or super-elastic characteristics. Shape memory characteristics may be simplistically described as follows. A metallic structure, for example, a Nitinol tube that is in an Austenitic phase may be cooled to a temperature such that it is in the Martensitic phase. Once in the Martensitic phase, the Nitinol tube may be deformed into a particular configuration or shape by the application of stress. As long as the Nitinol tube is maintained in the Martensitic phase, the Nitinol tube will remain in its deformed shape. If the Nitinol tube is heated to a temperature sufficient to cause the Nitinol tube to reach the Austenitic phase, the Nitinol tube will return to its original or programmed shape. The original shape is programmed to be a particular shape by well-known techniques.

Super-elastic characteristics may be simplistically described as follows. A metallic structure for example, a Nitinol tube that is in an Austenitic phase may be deformed to a particular shape or configuration by the application of mechanical energy. The application of mechanical energy causes a stress induced Martensitic phase transformation. In other words, the mechanical energy causes the Nitinol tube to transform from the Austenitic phase to the Martensitic phase. By utilizing the appropriate measuring instruments, one can determined that the stress from the mechanical energy causes a temperature drop in the Nitinol tube. Once the mechanical energy or stress is released, the Nitinol tube undergoes another mechanical phase transformation back to the Austenitic phase and thus its original or programmed shape. As described above, the original shape is programmed by well know techniques. The Martensitic and Austenitic phases are common phases in many metals.

Medical devices constructed from Nitinol are typically utilized in both the Martensitic phase and/or the Austenitic phase. The Martensitic phase is the low temperature phase. A material is in the Martensitic phase is typically very soft and malleable. These properties make it easier to shape or configure the Nitinol into complicated or complex structures. The Austenitic phase is the high temperature phase. A material in the Austenitic phase is generally much stronger than the material in the Martensitic phase. Typically, many medical devices are cooled to the Martensitic phase for manipulation and loading into delivery systems. When the device is deployed at body temperature, they return to the Austenitic phase.

Other materials that have shape memory characteristics may also be used, for example, some polymers and metallic composition materials.

The curved shape may also be formed in the distal end of the catheter 10, i.e. orienting member 50 using a passive component. Passive components are elements that cannot be shaped once introduced into the vasculature but are shapeable into a curved shape prior to introduction of the device into the vasculature. However, the passive components do substantially retain the curved shaped imparted to it even once introduced into the vasculature or body lumen. This may be done during the manufacturing process, or alternatively, by the clinician or a technician just prior to introduction of the delivery device into the vasculature.

Many of the components incorporated into the distal end of the catheter 10 may be designed as passive components to facilitate inducement of the curved shape. For example, in the case of a stent delivery system, the stent itself may be made or shaped with a curved shape prior to insertion into the vessel. In addition, the catheter inner body, guidewire lumen 14, including the main branch element, side branch element, or both elements, may be manufactured or subsequently shaped with a curved shape prior to introduction into the vessel.

In addition to the typical components that make up the delivery system described above, other components may be added to induce or facilitate the curved shape in the orienting member 50 or along the distal end of the catheter 10. In one embodiment of the invention, a biasing element is added to orienting member 50 to facilitate the curved shape. This element may be, for example, in the form of a stiffening wire, a ribbons or an extrusion. Still one of skill in the art would understand that other biasing element could be used.

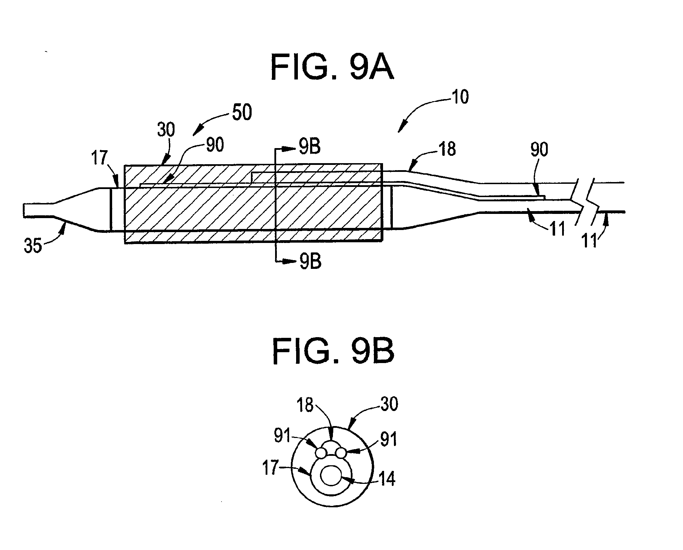

Figures 9A and 9B illustrated side and section views of a delivery device, catheter 10, having biasing elements 90 incorporated into orienting member 50 along the distal end 13 according to one embodiment of the present invention.

As can be seen, the passive delivery device has similar components as the delivery device 10 illustrated in Figures 1 through 4B, including an inner body guidewire lumen 14 for tracking over guidewire 33, an outer body catheter shaft 11, an expansion device (balloon) 17, a distal tip 35, and a secondary lumen 18 for the secondary or side branch guidewire 32. A medical device, stent 30, is also illustrated in these figures. However, the delivery device 10 illustrated in Figures 9A and 9B also has a pair of biasing wires 90 that start at the balloon 17 proximal seal and travel distally along orienting member 50 to a point just proximal the distal tip 35. The biasing wires 90 are capable of being bent and taking a set to form a curved shape along the orienting member 50.

It should be noted that Figure 9A shows the biasing wires 90 and the catheter 10 delivery system in a relatively straight configuration before the curved shape was induced upon the distal end. As earlier disclosed, the biasing wires 90 may be bent, for example, during manufacture, or by the clinician just prior to insertion into the vessel.

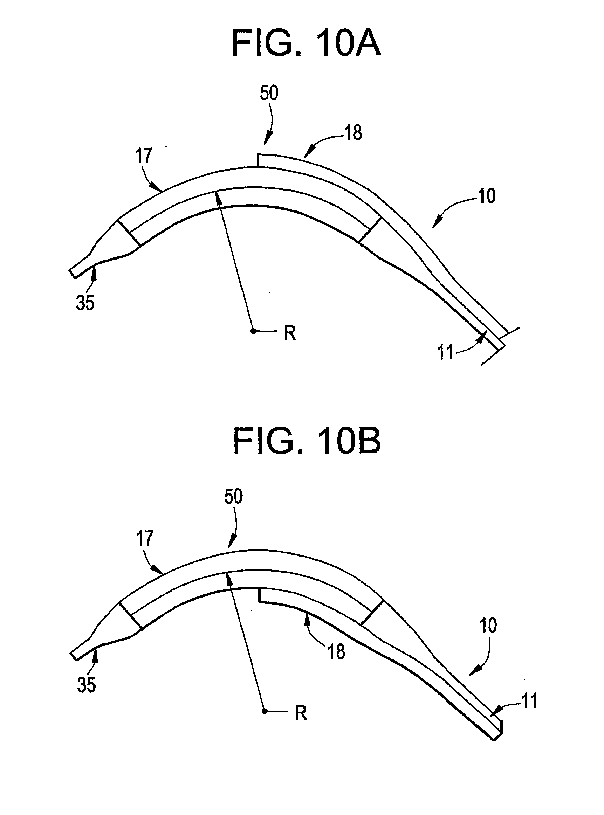

A catheter delivery device 10 having a curved shape with a radius R along the distal end is illustrated in Figures 10A and 10B. Figure 10A shows the delivery device having a curved shape with the secondary lumen 18 on the outside radius of the orienting member 50, also referred to as a greater curve bend. Figure 10B shows the delivery device having a curved shape with the secondary lumen 18 on the inside radius of the orienting member 50, which is also sometimes referred to as a lesser curve bend.

There are several methods that can be employed to passively induce the curved shape in the orienting member 50 along the distal end of the catheter delivery system 10, and are presented here for the purpose of example. One of skill in the art would understand that other methods might also be employed.

As discussed above, the curved shape may be set into the catheter delivery device 10 during the manufacturing process, or alternatively, by the clinician or a technician just prior to introduction of the delivery catheter 10 into the vasculature or body lumen.

The delivery catheter 10 and the components, particularly the orienting member 50, can be manufactured in a curved shape. Several different curved shapes may be made to allow a clinician to have a set from which to choose a delivery catheter 10 that has a curved shape that most closely resembles the actual vessel anatomy. Alternatively, the orienting member 50 may be appropriately shaped as needed.

In another embodiment of the invention the typical components comprising the distal end of the delivery catheter 10 are flexible. Auxiliary passive components having a predetermined curved shape, such as pre-bent biasing wires 91, are incorporated into the distal end and provide the curved shape for the orienting member 50. Alternatively, the pre-bend biasing wires 91 may be advanced or retracted through the inner body lumen 14 to provide a set shape to the flexible distal end portion of the catheter 10.

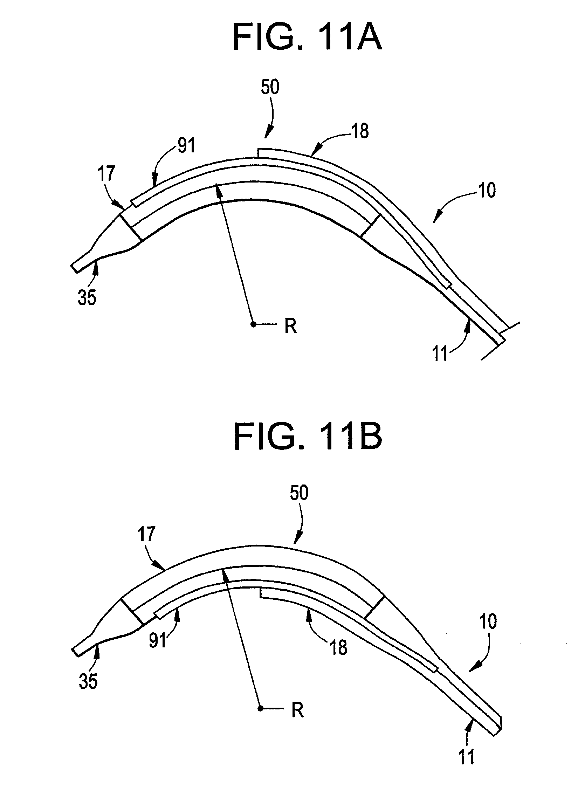

A catheter delivery device 10, including a pre-bent biasing wire inducing a curved shape with a radius R along orienting member 50 is illustrated in Figures 11A and 11B. Figure 11A shows the delivery catheter 10 with a curved shape with the secondary lumen 18 on the outside radius (greater curve bend) of the orienting member 50. Figure 11B shows the delivery device having a curved shape with the secondary lumen 18 on the inside radius (lesser curve bend) of the orienting member 50. In each case, pre-bent biasing wires 91 are incorporated into the orienting member 50, to provide the desired curved shape to the distal end portion of catheter 10.

In still another embodiment of the invention, one or more of the components comprising the distal end 13 of the delivery catheter 10 are made from materials that can be plastically deformed by the clinician prior to introduction of the catheter 10 into the vessel. This pre-delivery mechanical bend may be by actual manipulation and shaping by the clinician, with or without the use of a bending tool. Still other methods of bending could include specially designed packaging that will induce a bend onto the distal end of the delivery device when removed in a certain way. For example, the packaging may be designed to impart a convex (outside radius) bend for use in greater curve vessel anatomy if the delivery catheter 10 is removed from the packaging in one particular direction, and a concave (inside radius) bend for use in a lesser curve vessel anatomy if the delivery catheter 10 is removed a different direction.

In addition, the methods described for active bending after introduction into the vasculature may be used to induce a passive curved shape in the delivery catheter 10 or orienting member 50 before it is introduced into the anatomy.

The curved shape should be chosen such that the curvature of the device near the target payload region, i.e. orienting member 50, is approximately matched to that of vasculature anatomy. One method to determine the vascular anatomy is to insert the primary delivery guidewire 33 into the vessel and observe the curvature taken by the guidewire 33 relative to curvature of the vessel, or, in the case of bifurcated vessels, the ostium of the side branch vessel.

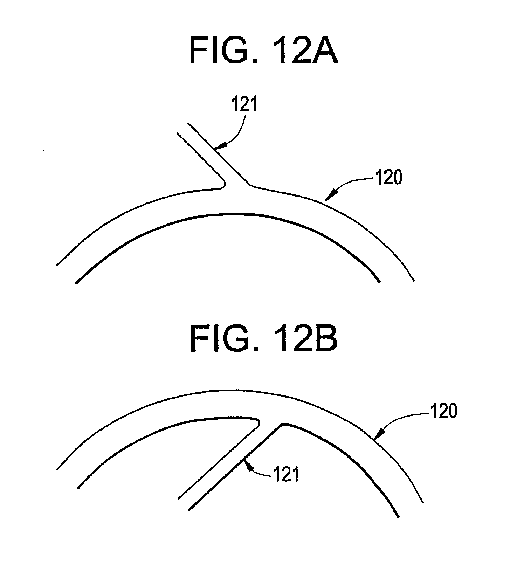

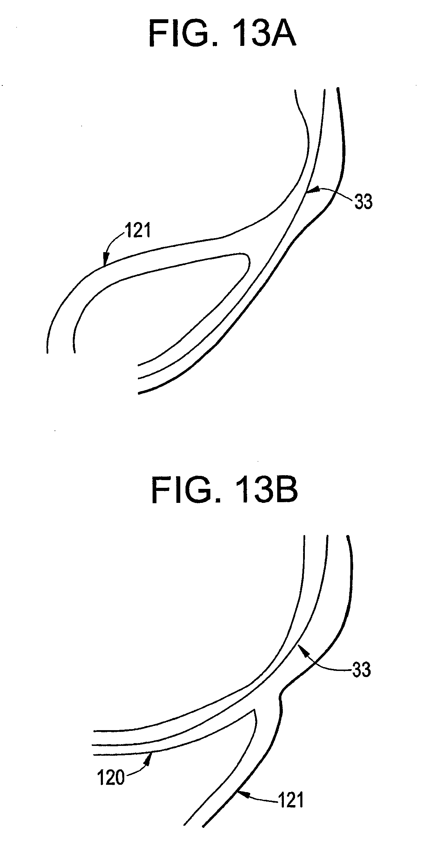

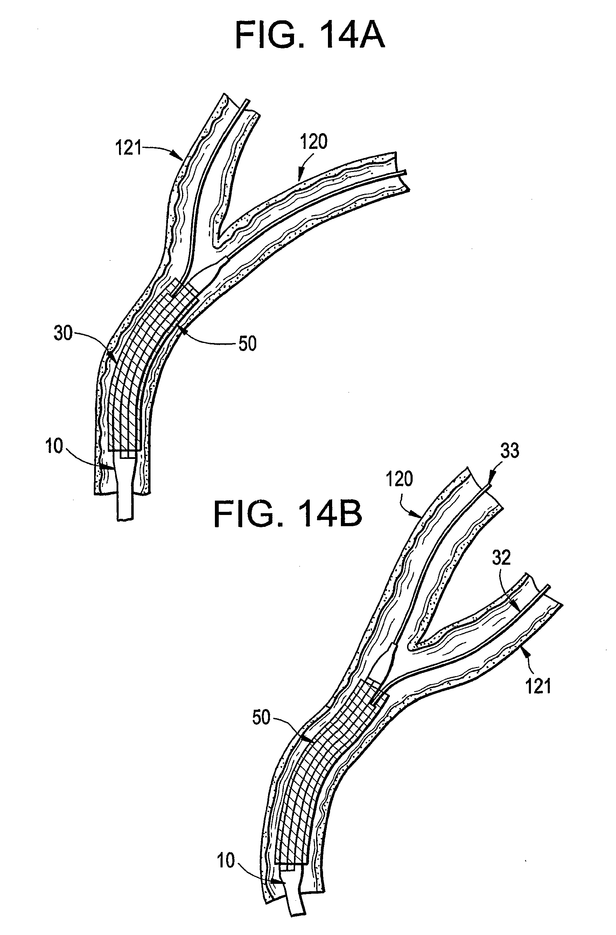

By way of example, Figures 12A illustrates a vessel anatomy having a main branch bend 120 and a side branch 121 off the greater curve (convex side) of the main branch bend 120. Figure 13A shows a guidewire 33 deployed in a similar main branch vessel 120 having a greater curve anatomy, and assists in viewing the vessel anatomy. To access the side branch 121 with a subsequently delivered second guidewire 32, the clinician would us a delivery catheter 10 with a greater curve side branch bend as shown in Figure 10A. The delivery catheter 10 is tracked over the primary guidewire 33 to the bifurcation location, and naturally rotates into position, with the secondary guidewire tube 18 facing the side branch vessel 121. The secondary guidewire 32 can then be advanced through the secondary guidewire tube 18 into the side branch vessel 121 as illustrated in Figure 14A. This ensures that the device orients fully to the side branch anatomy once both branches are wired and the device is seated at the carina of the bifurcation.

Similarly, Figure 12B illustrates a vessel anatomy having a main branch bend and a side branch off the lesser curve (concave side) of the main branch bend. A guidewire can be inserted into the main branch of the vessel to assist in viewing the vessel anatomy. Figure 13B shows a guidewire 33 deployed in a similar main branch vessel having a lesser curve anatomy. To access the side branch with a subsequently delivered second guidewire 32, the clinician would us a delivery catheter 10 with a lesser curve side branch bend as shown in Figure 10B. The delivery catheter 10. is tracked over the primary guidewire 33 to the bifurcation location, and naturally rotates into position, with the secondary guidewire tube 18 facing the side branch vessel 121. The secondary guidewire 32 can then be advanced through the secondary guidewire tube 18 into the side branch vessel 121 as illustrated in Figure 14B. This ensures that the device orients fully to the side branch anatomy once both branches are wired and the device is seated at the carina of the bifurcation.

Several factors may be considered when choosing or inducing a curved shape along the distal end of the delivery catheter system 10, particularly the location and shape of the curved shape, and the orientation of the side branch element to the curved shape.

Furthermore, is readily appreciable that implantation within the side branch 121 can be similarly achieved, owing to the versatility of the device. For example, as shown in Fig. 4B, by passing the main branch guide wire 33 through the orienting member 50 (with same positioned exterior to the payload), with the balloon 17 and side branch guidewire 32 within the payload (stent) 30, the device can be properly oriented by allowing the orienting member 50 to rotate the distal end of the catheter device 10 as the orienting member passes through the bend in the vessel. With this arrangement, the stent can be passed into the ostium of the side branch 121 and then deployed in the side branch 121.

It will be understood that this disclosure, in many respects, is only illustrative. Changes may be made in details, particularly in matters of shape, size, material, and arrangement of parts without exceeding the scope of the invention. Accordingly, the scope of the invention is as defined in the language of the appended claims.

## Claims

1. A device (10) for carrying a medically useful payload positioned upon the device to a location of interest relative to a non-linear path in a body lumen, comprising:
an elongate intralumenal member (11) having proximal and distal end portions (12, 13), and sized and dimensioned to travel to a location of interest in a body lumen; and
an orienting member (50) positioned along the distal end portion of the elongate intralumenal member, the orienting member exhibiting a curved shape and wherein a portion of the elongate intralumenal member proximal to the distal end of the elongate intralumenal member is constructed of a material of sufficiently high torsional flexibility such that the orienting member is rotatable about its longitudinal axis in response to the non-linear path relative to other parts of the device.

2. The device of claim 1 wherein the orienting member comprises a pre-shaped element.

3. The device of claim 2 wherein the pre-shaped element is the payload (30) or a biasing element (90).

4. The device of claim 3 wherein the biasing element is one of a shaped biasing wire (91), a shaped ribbon (134), a shaped extrusion, or an integral part of the elongate intralumenal member.

5. The device of claim 1 wherein the orienting member comprises a shapeable element.

6. The device of claim 5 wherein the shapeable element is the payload (30) or a plastically deformable element.

7. The device of claim 6 wherein the plastically deformable element is one of a wire, a ribbon, or an integral part of the elongate intralumenal member.

8. The device of claim 1 wherein the orienting member comprises an active component (61) capable of creating a curved shape.

9. The device of claim 8 wherein the active component comprises a hydraulically controlled element.

10. The device of claim 9 wherein the hydraulically controlled element includes at least one pressure chamber that is configured to induce a shape change in the orienting member corresponding to changes in pressure.

11. The device of claim 10 wherein the hydraulically controlled element includes an incompressible fluid or a compressible fluid.

12. The device of claim 8 wherein the active component comprises a mechanically controlled element that is configured to induce a shape change in the orienting member through the application of relative motion or force.

13. The device of claim 8 wherein the active component comprises an electrically controlled element that is configured to induce a shape change in the orienting member corresponding to a change in electrical potential or current.

14. The device of claim 13 wherein the electrically controlled element includes one of a piezoelectric element, a bimetallic strip with a least one resistive heating element, a conductive polymer that exhibits shape memory effects under the influence of electric potential, or an electro-mechanical actuator.

15. The device of claim 8 wherein the active component comprises a magnetically controlled element that is configured to induce a shape change in the orienting member corresponding to a change in magnetic field.

16. The device of claim 8 wherein the active component comprises a chemically controlled element that is configured to induce a shape change in the orienting member corresponding to a change in local chemistry.

17. The device of claim 8 wherein the active component comprises a thermally controlled element that is configured to induce a shape change in the orienting member corresponding to a change in temperature.

18. The device of claim 9 wherein the hydraulically controlled element includes at least one pressure chamber that changes shape corresponding to changes in pressure.

19. The device of claim 18 wherein the hydraulically controlled element includes at least one pressure chamber that changes shape corresponding to changes in chamber pressure.

20. The device of claim 19 wherein the hydraulically controlled element includes a compressible fluid.

21. The device of claim 8 wherein the active component comprises a mechanically controlled element that changes shape through the application of relative motion or force.

22. The device of claim 12 or claim 21 wherein the mechanically controlled element includes one of a pull wire, a deflectable tube, or a deflectable guidewire.

23. The device of claim 8 wherein the active component comprises an electrically controlled element that changes shape corresponding to a change in electrical potential or current.

24. The device of claim 23 wherein the electrically controlled element includes one of a piezoelectric transducer, a bimetallic strip, a resistive element or an electro-mechanical actuator.

25. The device of claim 8 wherein the active component comprises a magnetically controlled element that changes shape corresponding to a change in magnetic field.

26. The device of claim 15 or claim 25 wherein the magnetic field is an external magnetic field or an internal magnetic field.

27. The device of claim 15 or claim 25 wherein the magnetically controlled element includes a magnetostriction element.

28. The device of claim 27 wherein the magnetostriction element comprises Terfenol.

29. The device of claim 8 wherein the active component comprises a chemically controlled element that changes shape corresponding to a change in local chemistry.

30. The device of claim 16 or claim 29 wherein the change in local chemistry is caused by a chemical reaction.

31. The device of claim 30 wherein the chemical reaction causes swelling of the active component.

32. The device of claim 30 when dependent upon claim 16 wherein the chemical reaction causes a shape change of the active component.

33. The device of claim 30 when dependent upon claim 29 wherein the chemical reaction causes a change in stiffness of the active component.

34. The device of claim 32 or claim 33 wherein the change in local chemistry is caused by a change in chemical concentration.

35. The device of claim 32 or claim 33 wherein the change in chemical concentration causes swelling of the active component.

36. The device of claim 32 wherein the change in chemical concentration causes a shape change of the active component.

37. The device of claim 33 wherein the change in chemical concentration causes a change in stiffness of the active component.

38. The device of claim 8 wherein the active component comprises a thermally controlled element that changes shape corresponding to a change in temperature.

39. The device of claim 17 or claim 38 wherein the thermally controlled element includes regions having different coefficients of thermal expansion.

40. The device of claim 39 when dependent upon claim 17 wherein the thermally controlled element comprises nitinol.

41. The device of claim 8 wherein the active component comprises a thermally controlled element that changes mechanical properties corresponding to changes in temperature.

42. The device of claim 8 wherein the active component comprises a thermally controlled element that changes material phase corresponding to changes in temperature.

43. The device of claim 1 wherein the orienting member is a tube having a plastically deformable material embedded on or within the tube walls in preselected regions to impart curvature in the orienting member.

## Patentansprüche

1. Vorrichtung (10) zum Tragen einer medizinischen Nutzlast, die auf der Vorrichtung angeordnet ist, zu einer interessierenden Stelle relativ zu einem nichtlinearen Pfad in einem Körperlumen, umfassend:
ein langgestrecktes intraluminales Element (11) mit proximalen und distalen Endabschnitten (12, 13) und mit einer solchen Größe und Abmessungen, dass es sich zu einer interessierenden Stelle in einem Körperlumen bewegen kann; und
ein Orientierungselement (50), das entlang des distalen Endabschnitts des langgestreckten intraluminalen Elements angeordnet ist, wobei das Orientierungselement eine gebogene Gestalt aufweist und worin ein Abschnitt des langgestreckten intraluminalen Elements proximal zu dem distalen Ende des langgestreckten intraluminalen Elements aus einem Material mit ausreichend hoher Drehelastizität besteht, so dass das Orientierungselement als Reaktion auf den nichtlinearen Weg relativ zu anderen Teilen der Vorrichtung um seine Längsachse gedreht werden kann.

2. Vorrichtung nach Anspruch 1, worin das Orientierungselement ein vorgeformtes Element umfasst.

3. Vorrichtung nach Anspruch 2, worin das vorgeformte Element eine Nutzlast (30) oder ein Vorspannungselement (90) ist.

4. Vorrichtung nach Anspruch 3, worin das Vorspannungselement ein geformter Vorspannungsdraht (91), ein geformtes Band (134), eine geformte Extrusion oder ein integraler Bestandteil des langgestreckten intraluminalen Elements ist.

5. Vorrichtung nach Anspruch 1, worin das Orientierungselement ein formbares Element umfasst.

6. Vorrichtung nach Anspruch 5, worin das formbare Element die Nutzlast (30) oder ein plastisch verformbares Element ist.

7. Vorrichtung nach Anspruch 6, worin das plastisch verformbare Element ein Draht, ein Band oder ein integraler Bestandteil des langgestreckten intraluminalen Elements ist.

8. Vorrichtung nach Anspruch 1, worin das Orientierungselement eine aktive Komponente (61) umfasst, die eine gebogene Gestalt erzeugen kann.

9. Vorrichtung nach Anspruch 8, worin die aktive Komponente ein hydraulisch gesteuertes Element umfasst.

10. Vorrichtung nach Anspruch 9, worin das hydraulisch gesteuerte Element zumindest eine Druckkammer aufweist, die zur Auslösung einer Formveränderung in dem Orientierungselement in Übereinstimmung mit Druckveränderungen konfiguriert ist.

11. Vorrichtung nach Anspruch 10, worin das hydraulisch gesteuerte Element eine nicht komprimierbare Flüssigkeit oder eine komprimierbare Flüssigkeit aufweist.

12. Vorrichtung nach Anspruch 8, worin die aktive Komponente ein mechanisch gesteuertes Element umfasst, das zur Auslösung einer Formveränderung in dem Orientierungselement durch Aufbringen einer relativen Bewegung oder Kraft konfiguriert ist.

13. Vorrichtung nach Anspruch 8, worin die aktive Komponente ein elektrisch gesteuertes Element umfasst, das zur Auslösung einer Formveränderung in dem Orientierungselement in Übereinstimmung mit einer Veränderung des elektrischen Potentials oder Stroms konfiguriert ist.

14. Vorrichtung nach Anspruch 13, worin das elektrisch gesteuerte Element ein piezoelektrisches Element, einen bimetallischen Streifen mit zumindest einem Widerstandsheizelement, ein leitfähiges Polymer, das unter dem Einfluss eines elektrischen Potentials oder eines elektromechanischen Stellglieds Formgedächtniseffekte aufweist oder ein elektromechanisches Stellglied aufweist.

15. Vorrichtung nach Anspruch 8, worin die aktive Komponente ein magnetisch gesteuertes Element umfasst, das zur Auslösung einer Formveränderung in dem Orientierungselement in Übereinstimmung mit einer Magnetfeldveränderung konfiguriert ist.

16. Vorrichtung nach Anspruch 8, worin die aktive Komponente ein chemisch gesteuertes Element umfasst, das zur Auslösung einer Formveränderung in dem Orientierungselement in Übereinstimmung mit einer Veränderung der lokalen Chemie konfiguriert ist.

17. Vorrichtung nach Anspruch 8, worin die aktive Komponente ein thermisch gesteuertes Element umfasst, das zur Auslösung einer Formveränderung in dem Orientierungselement in Übereinstimmung mit einer Temperaturveränderung konfiguriert ist.

18. Vorrichtung nach Anspruch 9, worin das hydraulisch gesteuerte Element zumindest eine Druckkammer umfasst, die ihre Form in Übereinstimmung mit Druckveränderungen ändert.

19. Vorrichtung nach Anspruch 18, worin das hydraulisch gesteuerte Element zumindest eine Druckkammer umfasst, die ihre Form in Übereinstimmung mit Veränderungen des Kammerdrucks ändert.

20. Vorrichtung nach Anspruch 19, worin das hydraulisch gesteuerte Element eine komprimierbare Flüssigkeit aufweist.

21. Vorrichtung nach Anspruch 8, worin die aktive Komponente ein mechanisch gesteuertes Element umfasst, dass seine Form durch Aufbringen einer Relativbewegung oder Kraft verändert.

22. Vorrichtung nach Anspruch 12 oder Anspruch 21, worin das mechanisch gesteuerte Element einen Zugdraht, einen ablenkbaren Schlauch oder einen ablenkbaren Führungsdraht aufweist.

23. Vorrichtung nach Anspruch 8, worin die aktive Komponente ein elektrisch gesteuertes Element umfasst, das seine Form in Übereinstimmung mit einer Veränderung des elektrischen Potentials oder Stroms ändert.

24. Vorrichtung nach Anspruch 23, worin das elektrisch gesteuerte Element einen piezoelektrischen Wandler, einen bimetallischen Streifen, ein resistives Element oder ein elektromechanisches Stellglied aufweist.

25. Vorrichtung nach Anspruch 8, worin die aktive Komponente ein magnetisch gesteuertes Element umfasst, das seine Form in Übereinstimmung mit einer Magnetfeldveränderung ändert.

26. Vorrichtung nach Anspruch 15 oder Anspruch 25, worin das Magnetfeld ein externes Magnetfeld oder ein internes Magnetfeld ist.

27. Vorrichtung nach Anspruch 15 oder Anspruch 25, worin das magnetisch gesteuerte Element ein Magnetostriktionselement aufweist.

28. Vorrichtung nach Anspruch 27, worin das Magnetostriktionselement Terfenol umfasst.

29. Vorrichtung nach Anspruch 8, worin die aktive Komponente ein chemisch gesteuertes Element umfasst, das seine Form in Übereinstimmung mit einer Veränderung der lokalen Chemie ändert.

30. Vorrichtung nach Anspruch 16 oder Anspruch 29, worin die Veränderung der lokalen Chemie durch eine chemische Reaktion hervorgerufen wird.

31. Vorrichtung nach Anspruch 30, worin die chemische Reaktion eine Quellung der aktiven Komponente auslöst.

32. Vorrichtung nach Anspruch 30 in Abhängigkeit von Anspruch 16, worin die chemische Reaktion eine Formveränderung der aktiven Komponente hervorruft.

33. Vorrichtung nach Anspruch 30 in Abhängigkeit von Anspruch 29, worin die chemische Reaktion eine Veränderung der Steifheit der aktiven Komponente hervorruft.

34. Vorrichtung nach Anspruch 32 oder Anspruch 33, worin die Veränderung der lokalen Chemie durch eine Veränderung der chemischen Konzentration hervorgerufen wird.

35. Vorrichtung nach Anspruch 32 oder Anspruch 33, worin die Veränderung der chemischen Konzentration eine Quellung der aktiven Komponente verursacht.

36. Vorrichtung nach Anspruch 32, worin die Veränderung der chemischen Konzentration eine Formveränderung der aktiven Komponente verursacht.

37. Vorrichtung nach Anspruch 32, worin die Veränderung der chemischen Konzentration eine Veränderung der Steifheit der aktiven Komponente verursacht.

38. Vorrichtung nach Anspruch 8, worin die aktive Komponente ein thermisch gesteuertes Element umfasst, das seine Form in Übereinstimmung mit einer Temperaturänderung verändert.

39. Vorrichtung nach Anspruch 17 oder Anspruch 38, worin das thermisch gesteuerte Element Regionen mit unterschiedlichen thermischen Expansionskoeffizienten aufweist.

40. Vorrichtung nach Anspruch 39, in Abhängigkeit von Anspruch 17, worin das thermisch gesteuerte Element Nitinol umfasst.

41. Vorrichtung nach Anspruch 8, worin die aktive Komponente ein thermisch gesteuertes Element umfasst, das seine mechanischen Eigenschaften in Übereinstimmung mit Temperaturänderungen verändert.

42. Vorrichtung nach Anspruch 8, worin die aktive Komponente ein thermisch gesteuertes Element umfasst, das seine Materialphase in Übereinstimmung mit Temperaturänderungen verändert.

43. Vorrichtung nach Anspruch 1, worin das Orientierungselement ein Schlauch mit einem plastisch verformbaren Material ist, das auf oder in den Schlauchwänden in vorgewählten Regionen eingebettet ist, um dem Orientierungselement eine Biegung zu verleihen.

## Revendications

1. Dispositif (10) destiné à porter une charge médicalement utile positionnée sur le dispositif jusqu'à un site d'intérêt par rapport à un chemin non-linéaire dans une lumière corporelle, comportant :
un élément (11) intraluminal allongé présentant des sections (12, 13) d'extrémité proximale et distale, et dont la taille et les dimensions lui permettent de se déplacer à un site d'intérêt dans une lumière corporelle ; et
un élément (50) d'orientation positionné le long de la section d'extrémité distale de l'élément intraluminal allongé, l'élément d'orientation présentant une forme courbe et dans lequel une section de l'élément intraluminal allongé proximal par rapport à l'extrémité distale de l'élément intraluminal allongé est fabriquée dans un matériau d'une flexibilité en torsion suffisamment élevée de sorte que l'élément d'orientation peut tourner autour de son axe longitudinal en réponse au chemin non-linéaire par rapport à d'autres parties du dispositif.

2. Dispositif selon la revendication 1 dans lequel l'élément d'orientation comporte un élément préformé.

3. Dispositif selon la revendication 2 dans lequel l'élément préformé est la charge (30) ou un élément (90) de sollicitation.

4. Dispositif selon la revendication 3 dans lequel l'élément de sollicitation est un fil (91) de sollicitation qui a une forme, un ruban (134) qui a une forme, une extrusion qui a une forme, ou une partie intégrante de l'élément intraluminal allongé.

5. Dispositif selon la revendication 1 dans lequel l'élément d'orientation comporte un élément d'une forme modifiable.

6. Dispositif selon la revendication 5 dans lequel l'élément d'une forme modifiable est la charge (30) ou un élément plastiquement déformable.

7. Dispositif selon la revendication 6 dans lequel l'élément plastiquement déformable est un fil, un ruban, ou une partie intégrante de l'élément intraluminal allongé.

8. Dispositif selon la revendication 1 dans lequel l'élément d'orientation comporte un composant (61) actif apte à créer une forme courbe.

9. Dispositif selon la revendication 8 dans lequel le composant actif comporte un élément à commande hydraulique.

10. Dispositif selon la revendication 9 dans lequel l'élément à commande hydraulique comprend au moins une chambre de pression qui est configurée pour induire un changement de forme dans l'élément d'orientation correspondant à des changements de pression.

11. Dispositif selon la revendication 10 dans lequel l'élément à commande hydraulique comprend un fluide incompressible ou un fluide compressible.

12. Dispositif selon la revendication 8 dans lequel le composant actif comporte un élément à commande mécanique qui est configuré pour induire un changement de forme dans l'élément d'orientation par l'application d'un mouvement ou d'une force relative.

13. Dispositif selon la revendication 8 dans lequel le composant actif comprend un élément à commande électrique qui est configuré pour induire un changement de forme dans l'élément d'orientation correspondant à un changement de potentiel ou de courant électrique.

14. Dispositif selon la revendication 13 dans lequel l'élément à commande électrique comprend un élément piézoélectrique, une bande bimétallique comportant au moins un élément résistif chauffant, un polymère conducteur qui présente des effets de mémoire de forme sous l'influence d'un potentiel électrique, ou un actionneur électromécanique.

15. Dispositif selon la revendication 8 dans lequel le composant actif comporte un élément à commande magnétique qui est configuré pour induire un changement de forme dans l'élément d'orientation correspondant à un changement de champ magnétique.

16. Dispositif selon la revendication 8 dans lequel le composant actif comporte un élément à commande chimique qui est configuré pour induire un changement de forme dans l'élément d'orientation correspondant à un changement de chimie locale.

17. Dispositif selon la revendication 8 dans lequel le composant actif comporte un élément à commande thermique qui est configuré pour induire un changement de forme dans l'élément d'orientation correspondant à un changement de température.

18. Dispositif selon la revendication 9 dans lequel l'élément à commande hydraulique comprend au moins une chambre de pression qui change de forme en fonction des changements de pression.

19. Dispositif selon la revendication 18 dans lequel l'élément à commande hydraulique comprend au moins une chambre de pression qui change de forme en fonction des changements dans la chambre de pression.

20. Dispositif selon la revendication 19 dans lequel l'élément à commande hydraulique comprend un fluide compressible.

21. Dispositif selon la revendication 8 dans lequel le composant actif comporte un élément à commande mécanique qui change de forme par l'application d'un mouvement ou d'une force relative.

22. Dispositif selon la revendication 12 ou revendication 21 dans lequel l'élément à commande mécanique comprend un fil de traction, un tube susceptible de s'infléchir, ou un fil de guidage susceptible de s'infléchir.

23. Dispositif selon la revendication 8 dans lequel le composant actif comporte un élément à commande électrique qui change de forme en fonction d'un changement de potentiel ou de courant électrique.

24. Dispositif selon la revendication 23 dans lequel l'élément à commande électrique comprend un transducteur piézoélectrique, une bande bimétallique, un élément résistif ou un actionneur électromécanique.

25. Dispositif selon la revendication 8 dans lequel le composant actif comporte un élément à commande magnétique qui change de forme en fonction d'un changement de champ magnétique.

26. Dispositif selon la revendication 15 ou revendication 25 dans lequel le champ magnétique est un champ magnétique externe ou un champ magnétique interne.

27. Dispositif selon la revendication 15 ou la revendication 25 dans lequel l'élément à commande magnétique comprend un élément magnétostrictif.

28. Dispositif selon la revendication 27 dans lequel l'élément magnétostrictif comprend du Terfenol.

29. Dispositif selon la revendication 8 dans lequel le composant actif comporte un élément à commande chimique qui change de forme en fonction d'un changement de chimie locale.

30. Dispositif selon la revendication 16 ou la revendication 29 dans lequel le changement de chimie locale est provoqué par une réaction chimique.

31. Dispositif selon la revendication 30 dans lequel la réaction chimique provoque le gonflement du composant actif.

32. Dispositif selon la revendication 30 lorsque dépendante de la revendication 16 dans lequel la réaction chimique provoque un changement de forme du composant actif.

33. Dispositif selon la revendication 30 lorsque dépendante de la revendication 29 dans lequel la réaction chimique provoque un changement de rigidité du composant actif.

34. Dispositif selon la revendication 32 ou la revendication 33 dans lequel le changement de chimie locale est provoqué par un changement de concentration chimique.

35. Dispositif selon la revendication 32 ou revendication 33 dans lequel le changement de concentration chimique provoque le gonflement du composant actif.

36. Dispositif selon la revendication 32 dans lequel le changement de concentration chimique provoque un changement de forme du composant actif.

37. Dispositif selon la revendication 33 dans lequel le changement de concentration chimique provoque un changement de rigidité du composant actif.

38. Dispositif selon la revendication 8 dans lequel le composant actif comporte un élément à commande thermique qui change de forme en fonction d'un changement de température.

39. Dispositif selon la revendication 17 ou la revendication 38 dans lequel l'élément à commande thermique comprend des zones présentant différents coefficients d'expansion thermique.

40. Dispositif selon la revendication 39 lorsque dépendante de la revendication 17 dans lequel l'élément à commande thermique comporte du nitinol.

41. Dispositif selon la revendication 8 dans lequel le composant actif comporte un élément à action thermique qui change de propriétés mécaniques en fonction des changements de température.

42. Dispositif selon la revendication 8 dans lequel le composant actif comporte un élément à commande thermique qui change de phase matérielle en fonction des changements de température.

43. Dispositif selon la revendication 1 dans lequel l'élément d'orientation est un tube présentant un matériau plastiquement déformable incrusté sur les parois du tube ou incorporé à l'intérieur de ces dernières dans des zones présélectionnées qui est destiné à appliquer une courbure dans l'élément d'orientation.
